Europäisches Patentamt

European Patent Office

Office européen des brevets

(19)

(11) Numéro de publication: **0 201 407**
**B1**

(12) **FASCICULE DE BREVET EUROPEEN**

(45) Date de publication du fascicule du brevet: **18.07.90**

(51) Int. Cl.⁵: **A 61 F 2/36**

(21) Numéro de dépôt: **86400914.7**

(22) Date de dépôt: **24.04.86**

(54) **Jeu de pièces pour la réalisation d'une prothèse fémorale.**

(30) Priorité: **24.04.85 FR 8506214**

(43) Date de publication de la demande:
**12.11.86 Bulletin 86/46**

(45) Mention de la délivrance du brevet:
**18.07.90 Bulletin 90/29**

(84) Etats contractants désignés:
**BE CH DE FR GB IT LI LU NL SE**

(56) Documents cités:
**EP-A-0 017 743**
**EP-A-0 025 835**
**EP-A-0 038 897**
**EP-A-0 099 167**
**EP-A-0 163 121**

(73) Titulaire: **Montagne, Patrick**
**5, square du Lac Supérieur**
**F-78110 Le Vesinet (FR)**

(72) Inventeur: **Montagne, Patrick**
**5, square du Lac Supérieur**
**F-78110 Le Vesinet (FR)**

(74) Mandataire: **Plaçais, Jean-Yves et al**
**Cabinet Netter, 40, rue Vignon**
**F-75009 Paris (FR)**

EP 0 201 407 B1

**Description**

L'invention concerne le remplacement de l'articulation de hanche chez l'homme, par une prothèse fémorale du type comprenant:

— une tige fémorale destinée à être implantée dans le fémur, selon la direction générale du canal médullaire,

— une tête fémorale destinée à être logée dans le bassin, avec interposition éventuelle d'un cotyle prothétique, et

— un col fémoral reliant la tige et la tête.

La mise en place d'une prothèse articulaire comprend un temps de fixation des pièces dans l'os receveur, et un temps de reconstruction ou restauration de l'architecture articulaire.

L'invention s'intéresse plus particulièrement à ce temps de restauration de l'architecture articulaire. En effet, la qualité de celle-ci dépend des possibilités de débattement des pièces implantées l'une par rapport à l'autre, et des performances du bras de levier articulaire.

Ces deux éléments sont à leur tour sous la dépendance de l'orientation et de la longueur du col prothétique.

L'invention se propose d'améliorer la qualité de la fonction articulaire en augmentant les possibilités de variabilité, en longueur et en direction, du col prothétique.

Dans la plupart des prothèses fémorales réalisées jusqu'à présent, la direction du col est déterminée par la tige fémorale implantée dans l'os, ce col et la tige ne faisant qu'une seule et même pièce. La position du col par rapport à la tige est habituellement fixe. Ce réglage de la direction du col à partir de la tige n'offre que peu de souplesse; il faut d'ailleurs sacrifier une partie de l'os cortical fémoral, par un travail à la râpe, pour "offrir" au col son orientation.

Actuellement, la longueur du col est réglée:

— soit par l'emploi de prothèses monobloc à longueur de col variable, ceci augmentant considérablement le nombre de pièces prothétiques "lourdes",

— soit par la plus ou moins grande pénétration d'un cône morse cervical dans un cône morse intracéphalique. Mais, si ce système permet un réglage suffisant pour les grosses têtes (35/32mm), il devient insuffisant pour les petites (30/27mm) voire dangereux pour celles de petit diamètre en céramique. Ce réglage devient inexistant pour les très petites têtes de diamètre inférieur à 26mm.

— Il est également souhaitable de prévoir une orientation variable du col par rapport au plan frontal (plan vertical gauche-droite), c'est-à-dire un angle d'antéversion variable.

EP-A-38897 prévoit la possibilité de choisir l'antéversion après implantation de la tige dans le fémur. A cet effet, la pièce formant tige présente des trous répartis autour d'un axe d'assemblage, et une pièce de liaison peut être assemblée à la pièce formant tige dans différentes positions angulaires autour de l'axe d'assemblage, grâce à un pion pénétrant au choix dans l'un des trous.

Cet axe d'assemblage est confondu avec l'axe de la tige, de sorte que la tête fémorale solidaire de la pièce de liaison est toujours à la même hauteur et à la même distance horizontale par rapport à la tige. La prothèse décrite dans ce document ne permet donc pas d'atteindre les buts définis ci-dessus.

Ceux-ci sont atteints au contraire, selon l'invention, par un jeu de pièces comprenant au moins:

a) une pièce formant tige, définissant une tige fémorale présentant un axe et destinée à être implantée dans le fémur, selon la direction générale du canal médullaire,

b) une pièce formant tête définissant une tête fémorale destinée à être logée dans le bassin, avec interposition éventuelle d'un cotyle prothétique,

c) une première pièce de liaison définissant un col fémoral destiné à relier la tige fémorale et la tête fémorale,

caractérisé en ce que l'axe d'assemblage est oblique par rapport à l'axe de la tige, et s'oriente vers la tête fémorale.

De préférence, le jeu de pièces comprend en outre une seconde pièce de liaison définissant un col fémoral destiné à relier la tige fémorale et la tête fémorale et comportant des moyens pour son assemblage avec la pièce formant tige, en remplacement de la première pièce de liaison, de façon que la tête fémorale soit centrée sur l'axe d'assemblage.

L'invention vise également un jeu de pièces pour la réalisation d'une prothèse fémorale, ce jeu comprenant:

a) une pièce formant tige, définissant une tige fémorale présentant un axe et destinée à être implantée dans le fémur, selon la direction générale du canal médullaire, caractérisé en ce qu'il comprend au moins:

b) une première pièce de liaison définissant une tête fémorale destinée à être logée dans le bassin, avec interposition éventuelle d'un cotyle prothétique, et un col fémoral destiné à relier la tige fémorale et la tête fémorale,

la pièce formant tige et la première pièce de liaison comportant des moyens pour l'assemblage de cette dernière avec la pièce formant tige dans différentes positions autour d'un axe d'assemblage et la pièce de liaison étant conformée de façon qu'à ses différentes positions d'assemblage sur la pièce formant tige correspondent différents emplacements de la tête fémorale autour de l'axe d'assemblage.

Un tel jeu peut comprendre en outre une seconde pièce de liaison définissant une tête fémorale et un col fémoral destiné à relier la tige fémorale et la tête fémorale, et comportant des moyens pour son assemblage avec la pièce formant tige, en remplacement de la première pièce de liaison, de façon que la tête fémorale soit centrée sur l'axe d'assemblage.

Dans tous les cas, l'axe d'assemblage peut former avec l'axe de la tige un angle d'au moins 110°.

Selon un mode de réalisation, la tête fémorale présente une surface sphérique dont le centre est situé au voisinage de l'axe d'assemblage lorsque la prothèse est assemblée en utilisant la première pièce de liaison.

La pièce de liaison peut être assemblée à la pièce formant tige et le cas échéant à la pièce formant tige par des portées coniques d'angles petits.

Avantageusement, le jeu comprend au moins deux premières pièces de liaison et/ou au moins deux secondes pièces de liaison définissant des cols fémoraux de longueurs différentes.

L'invention sera mieux comprise grâce à la description donnée ci-après d'un exemple de réalisation non limitatif et aux dessins annexés, dans lesquels:

— la figure 1 représente un jeu de pièces selon l'invention;

— la figure 2 est une vue en coupe selon la ligne 2—2 de la figure 1;

— la figure 3 est une vue partielle en élévation de la pièce formant tige, où sont figurées schématiquement les différentes orientations possibles du col;

— la figure 4 est une vue selon la flèche B de la figure 3.

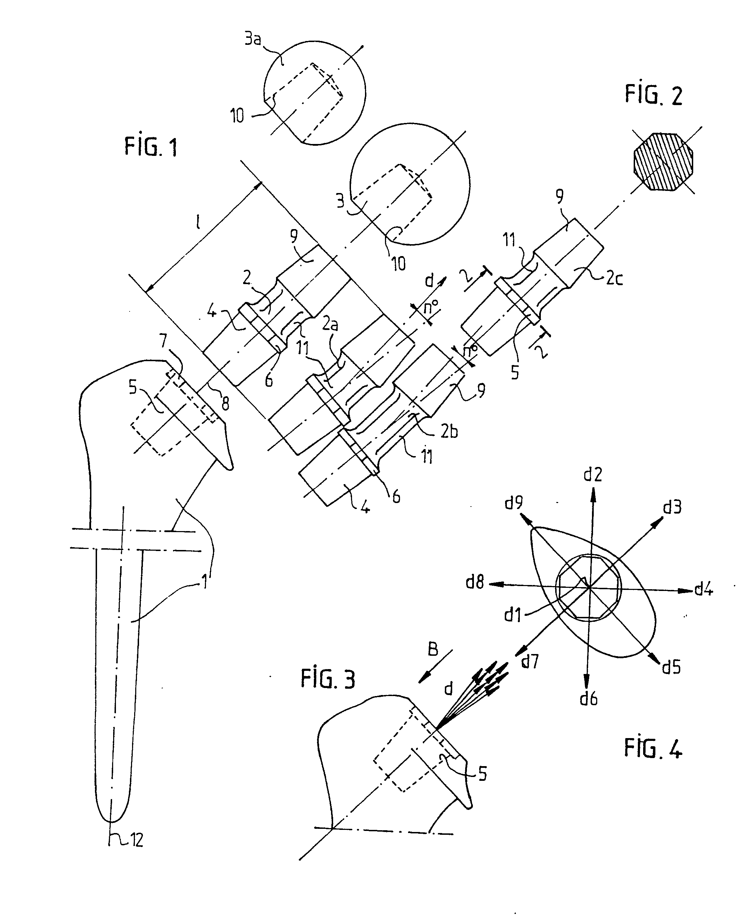

Le jeu de pièces représenté à la figure 1 comprend:

— une pièce formant tige fémorale 1 destinée à être implantée dans le canal médullaire du fémur;

— plusieurs pièces de liaison 2, 2a, 2b, 2c pouvant être assemblées au choix avec la pièce formant tige, et

— deux pièces formant tête fémorale 3 et 3a, pouvant être assemblées au choix avec chacune des pièces de liaison.

L'assemblage de la pièce formant tige 1 et de la pièce de liaison se fait par la coopération de portées coniques d'angle petit associées à des portées à facettes adjacentes aux grandes bases des cônes. Plus précisément, le cône mâle 4 de la pièce de liaison coopère par friction avec une cavité conique 5 de la pièce 1; l'indexation en rotation est obtenue par une partie prismatique 6 adjacente à la grande base du cône 4 de la pièce de liaison, s'engageant dans un élargissement prismatique conjugué 7 de la pièce 1. Les prismes 6 et 7 ont pour bases des polygones réguliers, dans l'exemple représenté des octogones (voir figure 2), de sorte que les portées à facettes qu'ils forment présentent une symétrie de répétition autour de l'axe 8 des cônes 4 et 5, lequel constitue l'axe d'assemblage, oblique par rapport à l'axe 12 de la tige.

Chaque pièce de liaison présente à son extrémité opposée au cône 4 un cône semblable 9 propre à coopérer avec une cavité conique 10 de la pièce formant tête 3 ou 3a, dont la surface extérieure est sphérique de façon à coopérer avec le cotyle sphérique, naturel ou artificiel, de la hanche. La partie prismatique 6 et la partie conique 9 de chaque pièce de liaison sont reliées entre elles par un corps intermédiaire 11 dont la longueur n'est pas la même pour toutes les pièces de liaison du jeu, la longueur totale 1 de la pièce de liaison pouvant ainsi prendre différentes valeurs. Par ailleurs, alors que les axes des cônes 4 et 9 sont confondus pour les pièces 2 et 2c, l'axe d du cône 9 des pièces 2a et 2b forme un petit angle de n°, par exemple 7,5°, avec l'axe d'assemblage.

Les figures 3 et 4 montrent comment, par le choix de la pièce de liaison et par sa position angulaire par rapport à la pièce formant tige, on peut faire varier l'orientation du col et par suite la position de la tête fémorale par rapport à l'axe d'assemblage, qui représente une direction moyenne du col. Le point dl de la figure 4 représente cette direction moyenne obtenue par l'utilisation d'une pièce de liaison droite telle que 2 ou 2c. Les flèches d2 à d9, réparties uniformément autour de l'axe 8, représentent huit orientations différentes correspondant aux huit positions possibles d'une pièce de liaison coudée telle que 2a ou 2b. On peut faire ainsi varier à la fois l'orientation en hauteur du col et l'antéversion. Comme le montre le faisceau de flèches de la figure 3, on obtient dans l'exemple représenté cinq orientations possibles en hauteur et pour chacune d'elles une, deux ou trois antéversions possibles (une flèche simple, deux flèches superposées ou trois flèches superposées).

Le choix de la longueur de la pièce de liaison détermine quant à lui la distance horizontale entre le centre de la tête et l'axe 12 de la tige. Le choix de la pièce formant tête détermine le diamètre de la tête fémorale sans affecter les autres paramètres.

Les cônes 4, 5, 9 et 10 sont de préférence des cônes morses.

Selon une variante non représentée, la pièce de liaison porte la tête fémorale de façon indissociable.

L'ensemble des pièces de liaison telles que représentées constitue un col modulaire variable qui peut s'orienter dans l'espace articulaire à reconstruire indépendamment de l'implantation de la tige.

Le col modulaire variable permet aussi, par son indépendance et sa longueur variable, d'utiliser des têtes de diamètres différents, en particulier des têtes en céramique de petite taille (28 mm) sans risque de fragilisation excessive.

Cette partie cervicale, appelée col modulaire variable, réalise une pièce prothétique légère, de faible volume, intermédiaire et indépendante. La longueur 1 et la direction d variables du col permettent un réglage amélioré de la reconstitution de l'architecture articulaire après la fixation dans les meilleures conditions des pièces "lourdes", tige fémorale et cotyle prothétique, dans leurs sites osseux récepteurs.

Le corps du col modulaire variable définit la longueur du col.

S'il est admis que cinq longueurs de col peuvent répondre aux différentes situations anatomiques possibles, ces cinq longueurs, en combinaison avec les neuf directions du col modulaire, réalisent quarante-cinq possibilités de positionnement de la tête fémorale prothétique dans l'espace articulaire à reconstruire.

Il existe deux types de pièces de liaison. Le premier, qui vient d'être décrit, est composé d'un corps et de deux extrémités inférieure et supérieure en forme de cône morse. Le deuxième comporte un seul cône morse inférieur, son autre extrémité supérieure étant usinée en forme de tête fémorale de petit diamètre (22 ou 26 mm par exemple).

Ce deuxième type peut être associé notamment à des cotyles d'un diamètre intérieur de 22 mm, le col porteur d'une tête de 26 mm servant à la mise en place d'une cupule intermédiaire pour implantation unipolaire et pour les reconstructions cotyloïdiennes par greffe osseuse.

Les avantages du col modulaire variable sont nombreux. Ils sont d'ordres technique, biomécanique, mécanique et économique.

Avantages Techniques

Au cours de l'acte opératoire, le chirurgien se trouve confronté à deux impératifs: le premier est d'obtenir la meilleure fixation primaire de la tige fémorale et du cotyle prothétique dans l'os; le deuxième est de restaurer l'architecture articulaire. L'utilisation actuelle d'un complexe cervico-diaphysaire monobloc oblige le chirurgien à anticiper l'orientation du col et ce, dès le premier temps de la fixation des prothèses dans l'os. Le col modulaire variable lui permettra de se décharger de cette préoccupation quant à la direction du col et à son débattement par rapport au cotyle déjà implanté. Il fera porter toute son attention sur la fixation (par scellement ou non) de la tige fémorale. Il tiendra ainsi pleinement compte du support osseux, de sa forme, de son volume et de ses orientations imposées souvent par des dismorphies congénitales ou acquises, post-traumatiques voire iatrogéniques en cas de reprise. L'os cortical fémoral sera ainsi exempté aussi d'un travail centro-médullaire d'abrasion à la râpe pour orienter directement le col.

Un avantage technique réside aussi dans la possibilité de reprises unipolaires en cas de descellement ou de migration de la pièce fémorale, et lorsque la conservation d'un cotyle bien fixé est souhaitée par le chirurgien (sujet âgé, mauvais état général. . . ). Dans de telles situations, l'utilisation du col modulaire permettra de mettre en place une nouvelle tête de diamètre adapté sans avoir ni à changer le cotyle, ni à remettre en place une prothèse du même type.

Un autre avantage technique se manifeste dans le cas de destruction cotyloidienne après ablation d'un implant scellé ou non. La mise en place d'une prothèse à col modulaire et cupule intermédiaire sur un lit de greffon permettra alors une reconstruction osseuse tout en permettant ultérieurement une nouvelle implantation cotyloïdienne sans avoir à changer la pièce fémorale intra osseuse.

Par ailleurs, lors des remplacements cervico-céphaliques en cas de fracture du col fémoral, la totalisation peut être réalisée sans abiation de la tige fémorale et en pouvant régler l'architecture articulaire.

Avantages Biomecaniques

Les avantages biomécaniques du col modulaire variable découlent de la possibilité de faire varier l'orientation en même temps que la longueur du col. On contrôle ainsi les deux facteurs de la qualité articulaire: le débattement, c'est-à-dire la mobilité, et le bras de levier, c'est-à-dire la force et la stabilité.

Le Debattement

La qualité de la fonction de la hanche prothétique dépend de la qualité du débattement global de la pièce fémorale par rapport à la pièce cotyloïdienne ou au cotyle osseux. Ce débattement global est sous la dépendance de trois facteurs intimement liés: le débattement mécanique théorique du col modulaire par rapport au bord du cotyle, le débattement anatomique permis par la position des pièces fixées dans l'os et le débattement permis par les structures molles péri-articulaires.

Le débattement théorique est le secteur angulaire balayé par l'axe du col dans un plan passant par le centrede la tête et du cotyle. Ce secteur d'angle est limité par le contact du col avec les bords axiaux du cotyle. Il sera d'autant plus important que le col sera plus petit et que la tête sera plus grosse. Par exemple, pour une même taille de tête, et de façon théorique, un col de section infiniment petit confinerait à un débattement de 180 degrés pour un cotyle hémisphérique. Au contraire, un col dont la section serait égale à la taille de la tête ne permettrait aucun débattement en dehors de la dislocation par effet de came sur les bords du cotyle. Un compromis est donc à trouver entre le diamètre de la tête, la grosseur du col et l'angle d'ouverture du cotyle.

Le débattement anatomique est sous la dépendance de la position des pièces fixées dans l'os receveur. Dans un plan horizontal les pièces sont habituellement antéversés pour être bien supportées par les os receveurs cotyloïdien et fémoral qui sont antéversés dans des conditions d'implantation ordinaires. Ainsi, dans un plan horizontal, le secteur angulaire couvert par l'axe du col sera plus important vers l'avant que vers l'arrière à partir de sa position de repos anatomique. De même, dans le plan frontal, un cotyle "couvrant-"dont le plan d'ouverture sera horizontalisé limitera l'abduction. Au contraire, un cotyle vertical libérera l'abduction.

La combinaison des deux débattements, frontal et horizontal, détermine la qualité de la fonction essentielle de la hanche: la flexion.

Le col modulaire variable permet d'harmoniser au mieux ce débattement anatomique par sa capacité, en pivotant sur son axe, de faire varier l'angle d'antéversion.

Le débattement physiologique fait intervenir les structures péri-articulaires et dépend de l'état de raideur préopératoire ainsi que de la voie d'abord choisie par le chirurgien. Là encore, le col modulaire variable permet de composer avec l'état pathologique et les habitudes chirurgicales.

### Le Bras de Levier

Défini par PAUWELS, il tient sous sa dépendance la charge qui s'exerce sur la tête fémorale prothétique. Sa valeur dépend directement de la distance entre le centre de la tête et le point d'application trochantérien des muscles fessiers. La charge exercée sur la tête est d'autant plus élevée que cette distance se trouve réduite. Il est donc très important de pouvoir moduler le bras de levier articulaire pour éviter les charges excessives et l'usure par détérioration du coefficient de friction du couple de frottement. Le col modulaire permet de placer de façon optimale la contre-réaction des fessiers en faisant varier la position du grand trochanter. Ceci est obtenu par la variation de l'angle cervico-diaphysaire et de la longueur du col, cette variation se réalisant sans phénomène d'allongement ou de raccourcissement du membre inférieur.

On répond ainsi aux conditions anatomiques de certaines dysmorphies: soit congénitales comme dans les dysplasies de hanche, soit acquises post-traumatiques voire iatrogéniques en cas de reprise avec perte du stock osseux.

### Avantages Mecaniques

Un avantage mécanique est à trouver dans la fixation intra-céphalique du col modulaire par un seul cône quel que soit le diamètre de la tête. Le corps du col assurant le réglage de la longueur, il n'y a pas fragilisation de la tête comme dans le cas du réglage de la longueur par pénétration plus ou moins profonde du cône morse dans la tête. Ceci est particulièrement important pour les têtes en céramique de petits diamètres qui semblent promettre une plus grande stabilité dans le temps au couple prothétique.

### Avantages Economiques

Les avantages économiques sont loin d'être négligeables. En effet, les matériaux modernes et performants qui entrent dans la fabrication des prothèses articulaires sont coûteux (titane, céramique...). Il est donc souhaitable, voire indispensable, de réduire au minimum la fabrication et l'immobilisation de pièces "lourdes" au profit de pièces "légères" élaborées à moindre frais.

La manutention, la manipulation et l'encombrement dus au stockage seront autant d'éléments d'amélioration dans le travail de l'équipe chirurgicale.

## Revendications

1. Jeu de pièces pour la réalisation d'une prothèse fémorale, ce jeu comprenant au moins:
   a) une pièce formant tige (1), définissant une tige fémorale présentant un axe (12) et destinée à être implantée dans le fémur, selon la direction générale du canal médullaire,
   b) une pièce formant tête (3, 3a) définissant une tête fémorale destinée à être logée dans le bassin, avec interposition éventuelle d'un cotyle prothétique, et
   c) une première pièce de liaison (2b) définissant un col fémoral destiné à relier la tige fémorale et la tête fémorale,
   la pièce formant tige et la première pièce de liaison comportant des moyens (5, 7, 4, 6) pour l'assemblage de cette dernière avec la pièce formant tige dans différentes positions autour d'un axe d'assemblage (8) et la pièce de liaison (2b) étant conformée de façon qu'à ses différentes positions d'assemblage sur la pièce formant tige correspondent différents emplacements de la tête fémorale autour de l'axe d'assemblage, caractérisé en ce que l'axe d'assemblage (8) est oblique par rapport à l'axe (12) de la tige, et s'oriente vers la tête fémorale.

2. Jeu selon la revendication 1, caractérisé en ce qu'il comprend au moins deux pièces formant tête (3, 3a) définissant des têtes de diamètres différents.

3. Jeu selon l'une des revendications 1 et 2, caractérisé en ce que la pièce de liaison et la pièce formant tête peuvent être assemblées par des portées coniques (9, 10) d'angle petit.

4. Jeu selon l'une des revendications précédentes, caractérisé en ce qu'il comprend en outre une seconde pièce de liaison (2) définissant un col fémoral destiné à relier la tige fémorale et la tête fémorale et comportant des moyens (4, 6) pour son assemblage avec la pièce formant tige, en remplacement de la première pièce de liaison (2b), de façon que la tête fémorale soit centrée sur l'axe d'assemblage (8).

5. Jeu de pièces pour la réalisation d'une prothèse fémorale, ce jeu comprenant:
   a) une pièce formant tige (1), définissant une tige fémorale présentant un axe (12) et destinée à être implantée dans le fémur, selon la direction générale du canal médullaire, caractérisé en ce qu'il comprend au moins:
   b) une première pièce de liaison définissant une tête fémorale destinée à être logée dans le bassin, avec interposition éventuelle d'un cotyle prothétique, et un col fémoral destiné à relier la tige fémorale et la tête fémorale,
   la pièce formant tige et la première pièce de liaison comportant des moyens pour l'assemblage de cette dernière avec la pièce formant tige dans différentes positions autour d'un axe d'assemblage (8) et la pièce de liaison étant conformée de façon qu'à ses différentes positions d'assemblage sur la pièce formant tige correspondent différents emplacements de la tête fémorale autour de l'axe d'assemblage, lequel est oblique par rapport à l'axe (12) de la tige, et s'oliente vers la tête fémorale.

6. Jeu selon la revendication 5, caractérisé en ce qu'il comprend en outre une seconde pièce de liaison définissant une tête fémorale et un col fémoral destiné à relier la tige fémorale et la tête fémorale, et comportant des moyens pour son assemblage avec la pièce formant tige, en remplacement de la première pièce de liaison, de façon que la tête fémorale soit centrée sur l'axe d'assemblage.

7. Jeu selon l'une des revendications précédentes, caractérisé en ce que l'axe d'assemblage (8) forme avec l'axe (12) de la tige un angle d'au moins 110°.

8. Jeu selon l'une des revendications précédentes, caractérisé en ce que la tête fémorale présente une surface sphérique dont le centre est situé au voisinage de l'axe d'assemblage lorsque la prothèse est assemblée en utilisant la première pièce de liaison.

9. Jeu selon l'une des revendications précédentes, caractérisé en ce que la pièce formant tige et la pièce de liaison peuvent être assemblées par des parties coniques (4, 5) d'angle petit.

10. Jeu selon la revendication 3 et la revendication 9, caractérisé en ce qu'au moins une pièce de liaison est terminée par deux cônes mâles (4, 9) pour l'assemblage avec la pièce formant tige et avec la pièce formant tête respectivement.

11. Jeu selon la revendication 10, caractérisé en ce que les cônes mâles de la première pièce de liaison ont des axes distincts formant entre eux un petit angle (n).

12. Jeu selon l'une des revendications précédentes, caractérisé en ce qu'il comprend au moins deux premières pièces de liaison (2a, 2b) et/ou au moins deux secondes pièces de liaison définissant des cols fémoraux de longueurs différentes.

**Patentansprüche**

1. Bausatz zum Aufbauen einer Femurprothese, wobei der Bausatz mindestens aufweist:
   a) ein schaftbildendes Teil (1), welches einen Femurschaft definiert, der eine Achse (12) aufweist und dazu bestimmt ist, entsprechend der allgemeinen Richtung des Knochenmarkkanals in den Femur eingesetzt zu werden,
   b) ein kopfbildendes Teil (3, 3a), welches einen Femurkopf definiert, der dazu bestimmt ist, unter möglichem Zwischenfügen einer prothetischen Gelenkpfanne im Becken gelagert zu werden, und
   c) ein erstes Verbindungsteil (2b), welches einen Femurhals definiert, der dazu bestimmt ist, den Femurschaft und den Femurkopf miteinander zu verbinden,
   wobei das schaftbildende Teil und das erste Verbindungsteil Mittel (5, 7, 4, 6) für den Zusammenbau des letzteren mit dem schaftbildenden Teil in unterschiedlichen Stellungen um eine Zusammenbauachse (8) herum aufweisen und wobei das Verbindungsteil (2b) derart geformt ist, daß seine unterschiedlichen Zusammenbaustellungen auf dem schaftbildenden Teil unterschiedlichen Standorten des Femurkopfs um die Zusammenbauachse herum entsprechen, dadurch gekennzeichnet, daß die Zusammenbauachse (8) bezüglich der Achse (12) des Schafts geneigt und in Richtung des Femurkopfs ausgerichtet ist.

2. Bausatz nach Anspruch 1, dadurch gekennzeichnet, daß er mindestens zwei kopfbildende Teile (3, 3a) enthält, welche Köpfe unterschiedlicher Durchmesser definieren.

3. 8ausatz nach einem der Ansprüche 1 und 2, dadurch gekennzeichnet, daß das Verbindungsteil und das kopfbildende Teil mittels konischer Bereiche (9, 10) mit kleinem Winkel zusammensetzbar sind.

4. Bausatz nach einem der vorhergehenden Ansprüche, dadurch gekennzeichnet, daß er zum Austausch gegen das erste Verbindungsteil (2b) außerdem ein zweites Verbindungsteil (2) aufweist, welches einen Femurhals definiert, der dazu bestimmt ist, den Femurschaft und den Femurkopf miteinander zu verbinden, und der Mittel (4, 6) für seinen Zusammenbau mit dem schaftbildenden Teil aufweist, derart daß der Femurkopf auf der Zusammenbauachse (8) zentrierbar ist.

5. Bausatz zum Aufbauen einer Femurprothese, wobei der Bausatz aufweist:
   a) ein schaftbildendes Teil (1), welches einen Femurschaft definiert, der eine Achse (12) aufweist und dazu bestimmt ist, entsprechend der allgemeinen Richtung des Knochenmarkkanals in den Femur eingesetzt zu werden, dadurch gekennzeichnet, daß er mindestens aufweist:
   b) ein erstes Verbindungsteil, welches einen Femurkopf und einen Femurhals definiert, wobei der Femurkopf dazu bestimmt ist, unter möglichem Zwischenfügen einer prothetischen Gelenkpfanne im Becken gelagert zu werden und wobei der Femurhals dazu bestimmt ist, den Femurschaft und den Femurkopf zu verbinden,
   wobei das schaftbildende Teil und das erste Verbindungsteil Mittel für den Zusammenbau des letzteren mit dem schaftbildenden Teil in unterschiedlichen Stellungen um eine Zusammenbauachse (8) herum aufweisen und wobei das Verbindungsteil derart geformt ist, daß seine unterschiedlichen Zusammenbaustellungen auf dem schaftbildenden Teil unterschiedlichen Standorten des Femurkopfes um die gegenüber der Achse (12) des Schafts geneigte und in Richtung des Femurkopfs ausgerichtete Zusammenbauachse herum entsprechen.

6. Bausatz nach Anspruch 5, dadurch gekennzeichnet, daß er zum Austausch gegen das erste Verbindungsteil außerdem ein zweites Verbindungsteil aufweist, welches einen Femurkopf und einen Femurhals definiert, der dazu bestimmt ist, den Femurschaft und den Femurkopf zu verbinden, und welches Mittel für seinen Zusammenbau mit dem schaftbildenden Teil aufweist, derart daß der Femurkopf auf der Zusammenbauachse zentrierbar ist.

7. Bausatz nach einem der vorhergehenden Ansprüche, dadurch gekennzeichnet, daß die Zusammenbauachse (8) mit der Achse (12) des Schafts einen Winkel von mindestens 110° einschließt.

8. Bausatz nach einem der vorhergehenden Ansprüche, dadurch gekennzeichnet, daß der Femurkopf eine sphärische Oberfläche aufweist, deren Mitte nach dem Zusammenbau der Prothese unter Benutzung des ersten Verbindungsteils in der Nähe der Zusammenbauachse angeordnet ist.

9. Bausatz nach einem der vorhergehenden Ansprüche, dadurch gekennzeichnet, daß das schaftbildende Teil und das Verbindungsteil mittels konischer Partien (4, 5) mit kleinem Winkel zusammensetzbar sind.

10. Bausatz nach Anspruch 3 und Anspruch 9, dadurch gekennzeichnet, daß mindestens ein Verbindungsteil durch zwei Konusfortsätze (4, 9) für den Zusammenbau mit dem schaftbildenden Teil bzw. mit dem kopfbildenden Teil begrenzt ist.

11. Bausatz nach Anspruch 10, dadurch gekennzeichnet, daß die Konusfortsätze des ersten Verbindungsteils unterschiedliche Achsen aufweisen, die zwischen sich einen kleinen Winkel (n) einschließen.

12. Bausatz nach einem der vorhergehenden Ansprüche, dadurch gekennzeichnet, daß er mindestens zwei erste Verbindungsteile (2a, 2b) und/oder mindestens zwei zweite Verbindungsteile aufweist, welche Femurhälse verschiedener Länge definieren.

## Claims

1. A set of parts for forming a femoral prosthesis, this set comprising at least:
a) a part forming a shaft (1) defining a femoral shaft having an axis (12) and intended to be implanted in the femur along the general direction of the medullary canal,
b) a part forming a head (3, 3a) defining a femoral head intended to be accommodated in the pelvis, with the possible interposition of a prosthetic cotyle, and
c) a first connecting part (2b) defining a femoral neck intended to join the femoral shaft and the femoral head,
the part forming the shaft and the first connecting part comprising means (5, 7, 4, 6) for the assembly of the latter with the part forming the shaft in different positions round an assembly axis (8) and the connecting part (2b) being shaped so that to its different assembly positions on the part forming the shaft, there correspond different fitted positions of the femoral head round the assembly axis, characterized in that the assembly axis (8) is inclined relative to the axis (12) of the shaft and is orientated towards the femoral head.

2. A set according to claim 1, characterized in that it comprises at least two parts forming heads (3, 3a) defining heads of different diameters.

3. A set according to one of claims 1 and 2, characterized in that the connecting part and the part forming the head can be fitted together by small-angled conical bearing surfaces (9, 10).

4. A set according to one of the preceding claims, characterized in that it comprises, moreover, a second connecting part (2) defining a femoral neck intended to join the femoral shaft and the femoral head and comprising means (4, 6) for its assembly with the part forming the shaft, replacing the first connecting part (2b), so that the femoral head is centred on the assembly axis (8).

5. A set of parts for forming a femoral prosthesis, this set comprising:
a) a part forming a shaft (1) defining a femoral shaft having an axis (12) and intended to be implanted in the femur along the general direction of the medullary canal, characterized in that it comprises at least
b) a first connecting part defining a femoral head intended to be accommodated in the pelvis, with the possible interposition of a prosthetic cotyle, and a femoral neck intended to join the femoral shaft and the femoral head, the part forming the shaft and the first connecting part comprising means for the assembly of the latter with the part forming the shaft in different positions round an assembly axis (8) and the connecting part being shaped so that to its different assembly positions on the part forming the shaft, there correspond different fitted positions of the femoral head round the assembly axis, which is inclined relative to the axis (12) of the shaft and is orientated towards the femoral head.

6. A set according to claim 5, characterized in that it comprises, moreover, a second connecting part defining a femoral head and a femoral neck intended to join the femoral shaft and the femoral head, and comprising means for its assembly with the part forming the shaft, replacing the first connecting part, so that the femoral head is centred on the assembly axis (8).

7. A set according to one of the preceding claims, characterized in that the assembly axis (8) forms an angle of at least 110' with the axis (12) of the shaft.

8. A set according to one of the preceding claims, characterized in that the femoral head has a spherical surface whose centre is situated near the assembly axis when the prosthesis is fitted together by using the first connecting part.

9. A set according to one of the preceding claims, characterized in that the part forming the head and the connecting part can be fitted together by small-angled conical parts (4, 5).

10. A set according to claim 3 and claim 9, characterized in that at least one connecting part ends in two male cones (4, 9) for the assembly with the part forming the shaft and with the part forming the head respectively.

11. A set according to claim 10, characterized in that the male cones of the first connecting part have separate axes forming a small angle (n) between them.

12. A set according to one of the preceding claims, characterized in that it comprises at least two first connecting parts (2a, 2b) and at least two second connecting parts defining femoral necks of different lengths.

FIG. 1

FIG. 2

FIG. 3

FIG. 4